# EUROPEAN PATENT SPECIFICATION

(11) **EP 3 161 153 B1**
(45) Date of publication and mention of the grant of the patent: **21.08.2019**
(21) Application number: 15736104.9
(22) Date of filing: 24.06.2015
(51) Int. Cl.: C12Q 1/689

(54) **METHOD AND REAGENTS FOR DETECTING WATER CONTAMINATION**
VERFAHREN UND REAGENZIEN ZUM NACHWEIS VON WASSERVERUNREINIGUNG
PROCÉDÉ ET RÉACTIFS PERMETTANT DE DÉTECTER LA CONTAMINATION DE L'EAU

(30) Priority: 30.06.2014 US 201462018834 P
(43) Date of publication of application: 03.05.2017
(73) Proprietor: Dow Global Technologies LLC, Midland, MI 48674 (US)
(72) Inventor: SCHOOK, Paul O., Midland, MI 48674 (US); YIN, Bei, Collegeville, PA 19426 (US)
(74) Representative: Houghton, Mark Phillip
(86) International application number: PCT/US2015/037329
(87) International publication number: WO 2016/028384

(56) References cited:
- WO-A2-02/077183
- DE-A1- 4 419 294
- US-A1- 2012 171 661
- STARNBACH M N ET AL: "SPECIES-SPECIFIC DETECTION OF LEGIONELLA-PNEUMOPHILA IN WATER BY DNA AMPLIFICATION AND HYBRIDIZATION", JOURNAL OF CLINICAL MICROBIOLOGY, vol. 27, no. 6, 1989, pages 1257-1261, XP002745357, ISSN: 0095-1137
- ULRICH REIDT ET AL: "Automated Immunomagnetic Processing and Separation of Legionella pneumophila with Manual Detection by Sandwich ELISA and PCR Amplification of the ompS Gene", JOURNAL OF LABORATORY AUTOMATION, vol. 16, no. 2, April 2011 (2011-04), pages 157-164, XP028161348, ISSN: 2211-0682, DOI: 10.1016/J.JALA.2010.07.003 [retrieved on 2010-08-06]
- GAIA VALERIA ET AL: "Consensus sequence-based scheme for epidemiological typing of clinical and environmental isolates of Legionella pneumophila", JOURNAL OF CLINICAL MICROBIOLOGY, AMERICAN SOCIETY FOR MICROBIOLOGY, US, vol. 43, no. 5, 1 May 2005 (2005-05-01), pages 2047-2052, XP002439888, ISSN: 0095-1137, DOI: 10.1128/JCM.43.5.2047-2052.2005 cited in the application

## Description

### BACKGROUND OF THE INVENTION

*Legionella pneumophila* is a ubiquitous bacterium associated with fresh water. This microorganism can cause a potentially fatal pneumonia, *i.e.,* Legionnaire's Disease, pneumonia, Pontiac fever, if inhaled; and thus is a serious problem for owners, operators, and treaters of domestic water systems, including operators of public facilities which provide water, such as hotels and restaurants, etc. While outbreaks of *L. pneumophila* infection associated with cooling water systems are rarer than outbreaks linked to domestic water systems, such as fountains and HVAC-related components, the ability of cooling towers to spread water droplets contaminated with *L. pneumophila* can cause an outbreak that covers a wide geographical area.

To provide guidance to owners, operators, and treaters of cooling systems various guidelines, codes of practices, and regulations have been put into place globally to detect and/or control the spread of Legionella in water sources. In North America, there are two guidelines written by industry groups that are soon to be finalized- the ASHRAE 188P and the CTI STD-159 standard. In addition, OSHA has recommended action levels for remediation and treatment based on counts of *Legionella* found in either domestic or industrial water systems.

The ability to identify and/or quantitatively identify *Legionella* from a mixed microbial population in a water source is critical to determine the potential for infections and to determine if the current treatment protocol is effective at either preventing or remediating *Legionella* in any water system. The US CDC has recommended a culture-based test approach that leverages the acid-tolerance of *Legionella,* a distinctive colony morphology, inherent resistance to the antibiotics glycine, vancomycin, polymixin B and cyclohexamide and the absolute requirements of addition iron and L-cysteine in growth media to successfully enrich *Legionella* bacteria from a mixed culture. However, *Legionella* is also a relatively slow-growing organism that requires 2-10 days under optimal growth conditions to appear on growth media. Given the complex media requirements to successfully isolate *Legionella* and the relatively lengthy incubation time needed for each growth step, this method for detecting *Legionella* is not optimal.

Other detection technologies take advantage of unique *Legionella* outer membrane proteins. A latex agglutination method by Oxoid Ltd, United Kingdom is a tool for confirming that a *Legionella* colony is in fact *L. pneumophila* SG1, the strain most linked to *Legionella* outbreaks. Other methods such as Biotica's LEGIPID™ test and Hydrosense's colorimetric antibody test, which has been marketed by Nalco as the Fastpath Duo System, are relatively rapid antibody-based detection methods for *Legionella* in water. For a direct visualization of *Legionella* in water there are fluorescent antibodies that delineate *Legionella* by serogroup 1 and 2-14/15/16 that can be used with a fluorescent microscope. A limitation of these methodologies and devices is that they require proteins to detect *Legionella.* As proteins can exist in water after cell death, these methods can result in false positive detections.

### SUMMARY OF THE INVENTION

In one aspect of the invention there is provided a reagent for examining a water supply for microbial contamination comprising a set of primers comprising a forward nucleotide sequence primer comprising SEQ ID NO: 4 and a reverse nucleotide sequence primer comprising SEQ ID NO: 10, the nucleotide sequences having high specificity for a *Legionella* microbial species, wherein said nucleotide sequences do not cross-react, or minimally cross- reacts, with a microbial species other than *Legionella.*

Preferably, the set of primers further comprises a forward primer of SEQ ID NOs: 1, 3, 5 or 6 and a reverse primer of SED ID NOs: 2, 7, 8 or 9. Preferably, the set of primers further comprise SEQ ID Nos: 1, 2, 3, 5, 6, 7, 8 and 9.

Preferably, the reagent comprises a substrate upon which one or more of the nucleotide sequences are immobilized or fixed. Preferably, the substrate is an array, a microarray, a microchip, a plastic surface, or a glass surface.

Preferably, the reagent includes a detectable label or label component that is associated with at least one primer.

In another aspect of the invention there is provided a kit comprising the reagent as hereinbefore described and one or more components selected from a label, a substrate, a label component capable of interacting with the label and generating a detectable signal.

In another aspect of the invention there is provided a water monitoring device comprising a reagent as hereinbefore described.

In another aspect of the invention there is provided a method of examining a water supply for microbial contamination comprising:
(i) contacting a water supply with a reagent according to any one of claims 1 to 5 in order to amplify a target sequence for a high degree of target specificity in polymerase chain reaction (PCR) or real time qualitative polymerase chain reaction (qPCR).methodologies; and
(ii) detecting, or measuring, a contaminating concentration of a *Legionella* species in the water supply.

Preferably, the PCR or qPCR comprises annealing the set of primers to a targeted nucleic acid sequence present in *Legionella* at a selected annealing temperature lower than 58° C.

Preferably, the method distinguishes between contamination with *Legionella* species from contamination with a *Pseudomonas* species.

In one aspect, a method of examining a water supply for microbial contamination is described which employs novel sensitive reagents having high specificity for a *Legionella* microbial species. This method employs reagents that do not cross-react, or minimally cross-reacts, with a microbial species other than *Legionella.* The method involves contacting a water supply with the reagent and detecting, or measuring, a contaminating concentration of a Legionella species in the water supply. In one embodiment, the method employs PCR or qPCR steps including annealing a reagent primer or a combination of primers to a targeted nucleic acid sequence present in Legionella at a selected annealing temperature. In another embodiment, the method employs hybridization based steps. The method permits the determination of whether Legionella concentration is within acceptable safety limits.

In another aspect, a novel reagent having high specificity for a *Legionella* microbial species, e.g., *L. pneumophila,* and which does not cross-react, or minimally cross-reacts, with a microbial species other than *Legionella* is provided. In one embodiment, the reagent comprises nucleotide primers, such as qPCR or PCR primers, with high specificity for multiple Legionella species, but not for *Pseudomonas* species. In another embodiment, the reagent includes a substrate upon which one or more of the nucleotide sequences are immobilized or fixed. In another embodiment, the reagent is a biosensor containing primers identified herein.

In yet another embodiment, a kit comprising one or more of the novel reagents described herein and other assay method components including labels, substrates, a label component capable of interacting with the label and generating a detectable signal, is provided.

Other aspects and advantages of these methods and compositions are described further in the following detailed description.

### DETAILED DESCRIPTION OF THE INVENTION

As described herein, novel compositions, e.g., reagents, and methods are provided that enable a fast and accurate detection of microbial contamination of *Legionella,* e.g., *Legionella pneumophila,* in water samples or supplies. As demonstrated in the examples below, the compositions (reagents and primers) and methods described herein provide an improvement in detection methods and compositions which have been used for *Legionella* detection. The reagents and primers described herein are highly specific to *Legionella,* and employ target sequences largely overlooked relative to more common detection genes such as *mip* or *dot*/*icm.* These primers are employed in methods of detection of contamination that employ qPCR-based detection of *Legionella* species, preferably *L. pneumophila* species, PCR and hybridization-based methodologies. The methods and the primers used therein allow lower temperatures to be employed in primer annealing, which provides efficiencies in energy use during performance of the methods. Further the reagents and methods described herein have less cross-reactivity against high levels of contaminating DNA, e.g., Pseudomonas, which is common in water systems, and these methods and compositions thereby accomplish the detection of *Legionella* contamination with less false positive results.

### A. Definitions and Components of the Methods and Compositions

By the term "water supply" or "samples" as used herein is meant any naturally occurring bodies of water, e.g., lakes, streams, rivers, or industrial or domestic artificial container or body carrying water, e.g., reservoirs, pools, fountains, potable or drinking water, HVAC systems containing or carrying water, bottled water, industrial water supplies or containers, waste water containers, etc. These water supplies or samples can contain purely *Legionella,* a mixed population of microbial organisms with high *Legionella* levels, a mixed population of microbial organisms with low *Legionella* levels, a mixed population of microbial organisms with no *Legionella,* or no microbial contamination. Such samples or water supplies can contain unconcentrated or concentrated DNA samples of a target sequence from the indicated microorganism.

By "target" is meant a nucleic acid sequence that is found in one or more Legionella species and has certain desirable characteristics. Such characteristics include relatively stable expression at relatively high levels compared to other Legionella gene sequences. Another characteristic is that the nucleotide sequence expression is insensitive to growth conditions and sufficiently unique to *Legionella* to prevent detection of non-Legionella microorganisms. The target sequence may be found in nucleic acid materials from the Legionella microorganisms that infect mammalian subjects, e.g., humans, and contaminate water or water supplies. In one embodiment, the target is a sequence encodes portions of the Legionella major outer membrane protein. The target is a single-stranded ribonucleic acid sequence, or a single strand of a deoxyribonucleic acid sequence from Legionella. Included in this definition are RNA, mRNA, microRNA, a single strand of DNA, cDNA, small-interfering RNA (siRNA), short-hairpin RNA (shRNA), peptide nucleic acid (PNA), transfer RNA (tRNA), ribosomal RNA (rRNA) and DNA from a Legionella gene. In one embodiment, the selected target gene is known as the major outer membrane protein gene of Legionella, or *ompS, omp28, lpg1974* or *lpnomp28.* In one embodiment, the target, *ompS,* is a gene that appears to be stably expressed regardless of growth condition and is heavily conserved within the *Legionella* genus, making it an appealing target for detection of water supply contamination. The nucleotide sequence of the *ompS* gene can be found in the Genbank datdabase at accession No. M76178. See also, SEQ ID NO: 17. This target gene also includes the *mompS* or major outer membrane protein precursor gene, the sequences of which is publically available in the Genbank database at accession No. AF078147.

More specifically, the target of the ompS sequences may be nucleic acid regions of SEQ ID NO: 17, or a homolog or naturally occurring ortholog of same that provides a suitable "target" for detection by a nucleic-acid based detection tool, such as qPCR primers, PCR primers or hybridization probes. For ease of use in identifying target regions of ompS, this specification will refer to regions of SEQ ID NO: 17. However, it is understood by one of skill in the art that homologous sequences of Legionella ompS genes having modifications or mutations from the sequence of SEQ ID NO: 17 or from other Legionella species ompS are also included in the descriptions of the reagents and methods herein.

By "Legionella" is meant any species of the genus Legionella, particularly those species commonly found in bodies of water or water supplies. Legionella species include without limitation, *L. pneumophila, L. dumoffii, L. longbeachae, L. cherrii, L. pneumophila* SG6A, *L. pneumophila* SG6B, *L. feelei* or combinations thereof. It is a goal of the reagents and methods described herein to detect contaminating *L. pneumophila,* among other species, because it causes human disease.

The term "reagent" as used herein can refer to a single primer, one or multiple sets of forward and reverse primers, labeled primers, primers immobilized on a substrate or in an array or microarray, or devices incorporating any of the above for use in detecting Legionalla contamination in various water supplies.

The term "primer" as used herein is intended to mean oligonucleotide sequences that can bind to and amplify the target sequence in the performance of PCR or qPCR. In one embodiment, a primer set comprises a forward primer that binds to the coding strand of the target in the 5' to 3' direction. In another embodiment, a primer set comprises a reverse primer that binds to the complement of the target sequence in the 3' to 5' direction. As used herein the primers may be about 15 to about 50 nucleotides in length, including any intermediate length in-between, including at least 15, 16, 17, 18, 19, 20, 21, 22, 23, 24, 25, 26, 27, 28, 29, 30, 31, 32, 33, 34, 35, 36, 37, 39, 40, 41, 42, 43, 44, 45, 46, 47, 48, 49 or 50 nucleotides in length. Such primers may be employed in solution, e.g., in buffer, or immobilized on a substrate or microarray. In other embodiment, the primers may be labeled with detectable, e.g., fluorescent, labels, e.g., according to the TaqMan® methodology so that the target-specific oligonucleotides produce a fluorescent signal only when the target DNA is amplified during qPCR, or using fluorescent or other labels such as SYBR® Green I dye.

The term "microarray" refers to an ordered arrangement of hybridizable array elements. In one embodiment, a microarray comprises polynucleotide probes that hybridize to the specified target sequence, on a substrate. In another embodiment, a microarray comprises multiple primers (reverse and forward), optionally immobilized on a substrate.

The term "PCR array" refers to microfluidics card or multiwell plate containing an ordered arrangement of gene-specific forward and reverse primers and fluorescence-labeled probe in each micro-well. This array is used with the sample from the water supply, or a product derived therefrom (e.g., cDNA or cRNA derived from RNA) in real-time PCR reactions which are monitored using fluorescent dye, for example SYBR Green or Taqman technology using a reporter fluorescent dye. Each set of primers is designed to amplify the target sequence of interest, such as those described herein. Such primers can readily be designed by one of skill in the art using known techniques, given the instructions on the primer identities described in this specification. See e.g., reference 13. Primers may also be prepared or available commercially, for example from Invitrogen: http://bioinfo.invitrogen.com/genome-database/browse/gene-expression/keyword/Taqman%20primers?ICID=uc-gex-Taqman.

The term "real-time quantitative PCR", or "qPCR" refers to a technique combining PCR amplification and detection into a single step. With qPCR, fluorescent dyes are used to label PCR products during thermal cycling. Real-time PCR instruments measure the accumulation of fluorescent signal during the exponential phase of the reaction for fast, precise quantification of PCR products and objective data analysis. qPCR reaction products are fluorescently labeled using one of two main strategies: Reactions are run in real-time PCR instruments with thermal cycling and fluorescence detection capabilities. By using highly efficient PCR primers and optimal conditions for amplification, every target molecule is copied once at each cycle and data are captured throughout the thermal cycling. Since qPCR reactions are set up with a large molar excess of PCR primers and thermostable DNA polymerase, in the early rounds of thermal cycling, target-template is the limiting factor for the reaction thus, fluorescent signal is directly proportional to the amount of target in the input sample.

### B. Reagents of the Disclosure

In one embodiment, a reagent for examining a water supply for microbial contamination has high specificity for a *Legionella* microbial species, and does not cross-react, or minimally cross-reacts, with a microbial species other than *Legionella.* In one embodiment, such a reagent comprises at least one nucleotide sequence primer (DNA or RNA) that amplifies or hybridizes to a target sequence in Legionella. In another embodiment the target sequence is found between and including nucleotides 528 and 844 of SEQ ID NO: 17. In one embodiment, the ompS target sequence is found between and including nucleotides 327 and 491 of SEQ ID NO: 17. In another embodiment the target sequence is found between and including nucleotides 327 and 844 of SEQ ID NO: 17. In another embodiment the target sequence is found between and including nucleotides 327 and 491 of SEQ ID NO: 17. In another embodiment the target sequence is found between and including nucleotides 327 and 550 of SEQ ID NO: 17. In another embodiment the target sequence is found between and including nucleotides 327 and 680 of SEQ ID NO: 17. In another embodiment the target sequence is found between and including nucleotides 528 and 550 of SEQ ID NO: 17. In another embodiment the target sequence is found between and including nucleotides 528 and 680 of SEQ ID NO: 17. In another embodiment the target sequence is found between and including nucleotides 660 and 844 of SEQ ID NO: 17. In another embodiment the target sequence is found between and including nucleotides 660 and 680 of SEQ ID NO: 17. In still other embodiments, the target regions may include those regions identified above including from about 5 to about 15 additional nucleotides on either end of the identified sequences.

These reagents may be primers as defined above which are selected from the forward primer sequences set out in Table 1 below as SEQ ID NOs: 1, 3, 4, 5 and 6. It should be understood that these sequences may differ from those of Table 1 by certain modifications. In one embodiment, a modification is indicated by the presence of the indicated wobble bases shown and defined in Table 1. Other modifications of the primer sequences of Table include primer sequences that differ from those of Table 1 by including from 1 to about 5 additional contiguous nucleotides 5' to the 5' nucleotide base of each forward primer location in SEQ ID NO: 17. In another embodiment additional modified primer sequences may differ from those of Table 1 by including from 1 to about 5 additional contiguous nucleotides 3' to the 3' nucleotide base of each forward primer located in SEQ ID NO: 17. Data [not shown] illustrates that, one can see how the identified primers may be modified by adding contiguous bases to either end of the identified primer, or deleting one or more bases from either end of the primer, or a combination of both types of modifications. In yet a further embodiment, a primer may differ from a sequence of Table 1, by deleting 1, 2, 3, 4, or 5 bases from the 5' or 3' end of the related sequence in Table 1 and adding 1, 2, 3, 4, or 5 additional contiguous bases to the opposite end of the primer, thus shifting the primer sequence. For example, one primer sequence could be a modification of SEQ ID NO:4 and span nucleotides 526 to 550 of SEQ ID NO: 17 or 526 to 547 of SEQ ID NO: 17 and so on.

These reagents may be primers as defined above which are selected from the reverse primer sequences set out in Table 1 below as SEQ ID NOs: 2, 7, 8, 9, 10. It should be understood that these sequences may differ from those of Table 1 by modifications such as the indicated wobble bases in that table. As described above, additional modifications can result in modified reverse primer sequences that differ from those of Table 1 by including from 1 to about 5 additional contiguous nucleotides 5' to the 5' nucleotide base of each reverse primer in SEQ ID NO: 17. In another embodiment additional reverse primer sequences may differ from those of Table 1 by including from 1 to about 5 additional contiguous nucleotides 3' to the 3' nucleotide base of each reverse primer in SEQ ID NO: 17. In a manner analogous to that described above for the forward primers, the reverse primers of Table 1 may be modified by shifting the primer sequence 1 to 5 bases 5' or 3' from the primers position on SEQ ID NO: 17.

Thus, in one embodiment, the reagent comprises a set of primers comprising a forward nucleotide sequence primer and a reverse nucleotide sequence primer selected from Table 1. In one embodiment, the reagent contains a forward primer comprising SEQ ID NO:4 or SEQ ID NO:4 and of at least one of SEQ ID NO: 1, 3, 5 or 6 and the reverse primer comprises SEQ ID NO:10 or SEQ ID NO:10 and at least one of SEQ ID NO: 2, 7, 8, and 9. Possible combinations, disclosed but not claimed are, the reagent contains the forward primer comprising SEQ ID NO: 4 and the reverse primer comprising one of SEQ ID NO: 8 or 9. In a similar manner, the reagent may comprise other combinations of the forward and reverse primers of Table 1, SEQ ID NOs: 1-10. Also disclosed the reagent comprises multiple sets of reagents selected from the primers comprising SEQ ID NOs: 1 - 10 or 3 - 10. Also disclosed, the reagent may_comprise all nucleotide sequence primers selected from primer sequences comprising SEQ ID NO: 1-10, or sequences having modifications thereof on the 5' or 3' ends as described above.

In still another embodiment, a reagent comprises a substrate upon which one or more of the nucleotide sequences are immobilized or fixed. Such a substrate is an array, a microarray, a microchip, a plastic surface, or a glass surface. Association of the primer on the substrate and methods for accomplishing the association are well known in the art. Alternatively, the primer sequences may be provided in a suitable buffer depending upon the type of method in which the reagent is to be used.

In still another embodiment, the reagent includes a primer to which a detectable label or label component is associated. Suitable detectable labels include fluorescent labels such as those employed by the TAQMAN reagents, or other known fluorescent molecules. Suitable labels may be selected from among many known label types by one of skill in the art given the teachings of this specification.

In yet another embodiment, a reagent can be in the form of a kit containing one or more of the primers, reverse and forward primer sets or labeled primer-probes, suitable labels and labeling methodologies, suitable substrates, and/or label component, e.g., enzymes, capable of interacting with the label associated with a primer and generating a detectable signal.

In yet another embodiment, the reagent is configured as a water monitoring device comprising the primers described herein.

### C. Methods of Detecting Contamination

The reagents, e.g., primers described herein, are desirably used in methods for Legionella detection involving the techniques of polymerase chain reaction (PCR), quantitative PCR (qPCR), or oligonucleotide hybridization-based methodologies.

In one aspect, a method of examining a water supply for microbial contamination, particularly contamination with Legionella, is provided. Such a method involves contacting a water supply with a reagent having high specificity for a *Legionella* microbial species, wherein the reagent does not cross-react, or minimally cross-reacts, with a microbial species other than *Legionella.* Thereafter, the method involves detecting, or measuring, a contaminating concentration of a Legionella species in the water supply.

Also described is that the reagent employed in the method may be one of the reagents described herein. For example, the method can employ a reagent comprising at least one nucleotide sequence primer selected from:
(a) forward primer sequence 327 comprising SEQ ID NO: 3
(b) forward primer sequence 528 comprising SEQ ID NO: 4
(c) forward primer sequence 660 comprising SEQ ID NO: 5;
(d) forward primer sequence 825 comprising SEQ ID NO: 6
(e) reverse primer sequence GC- 327 comprising SEQ ID NO: 7;
(f) reverse primer sequence GC- 528 comprising SEQ ID NO: 8
(g) reverse primer sequence GC- 660 comprising SEQ ID NO: 9;
(h) reverse primer sequence GC- 825 comprising SEQ ID NO: 10; or
(i) a combination of primer sequences comprising (a) through (h) or
(j) modifications of such primer sequences with wobble bases or by adding or deleting one or more 5' or 3' bases or by shifting the position of the primer 1 to 5 bases along its position in SEQ ID NO: 17.

In another embodiment, the method employs multiple sets of reagents selected from the primers of (b) and (h) and (a), (c) through to (g), or includes multiple steps using some or all of the primers or reverse/forward primer pairs in a suitable methodology. Any of the reagents described above are believed to be useful in the methods described below and in the examples.

Thus, in one embodiment, the method involves performing PCR or qPCR steps to amplify the target sequence by annealing a primer or a combination of primers to the targeted nucleic acid sequence present in Legionella at a selected annealing temperature. In one embodiment, the annealing temperature is less than 58°C. In another embodiment, the annealing temperature is about 40°C. In another embodiment, the annealing temperature is about 41°C. In another embodiment, the annealing temperature is at least 40, 41, 42, 43, 44, 45, 46, 47, 48, 49, or 50°C. In still other embodiments, the annealing temperature of the reagents described herein is lower than the annealing temperatures employed by use of other known ompS primers. For example, as shown in Table 2, the method can involve contacting a sample with 1µM primer for 3 min at 95°C; and performing 35 cycles of denaturation for 30 sec at 94°C; annealing the primers for 30 sec at 40°C; elongation for 30 sec at 72°C; and a 10 min 72°C extension step. Still other conditions are described in detail in the examples below.

In another embodiment, the measuring comprises hybridizing a primer to a targeted nucleic acid sequence present in Legionella and performing a polymerase chain reaction (PCR) or a quantitative polymerase chain reaction (qPCR). These methods employ amplifying the targeted sequence and determining whether said Legionella concentration is within acceptable safety limits.

A variety of methods of measuring or detecting the amount of contamination are known in the art once the primers have amplified the target sequence or identified in by hybridization. Methods of detecting can include running the amplified or hybridized target sequences on with agarose gel for visualization. Running electric current through the gel causes the negatively-charged DNA to migrate to positively charged side of gel. The gel is stained with dye that binds to double-stranded DNA, thereby allowing visualization. The larger the DNA, the slower it moves through the gel. Still other methods of measuring the results of PCR and hybridization methodologies can be selected by one of skill in the art.

Thus, in one embodiment, the methods employ a reagent with high specificity for multiple Legionella pneumophila only. Thus, in one embodiment, the method and reagents can distinguish between contamination of a water supply with Legionella from contamination with a *Pseudomonas* species, such as *Pseudomonas aeruginosa* or *Pseudomonas denitrificans.*

As demonstrated in the examples below, the primers described herein provide a higher degree of target specificity in qPCR and PCR methodologies. The examples showed testing against purified DNA from various strains, and testing against DNA extracted from six CDC ELITE testing samples. As discussed below, primers 528/825gc are better than the known SEQ ID NO: 15 and 16 primer sequences described in US patent publication No. 20120171661 because they have decreased cross-reactivity with other species of microorganism. These qPCR primers, e.g., SEQ ID NO: 1 and 2 or 4 and 10 allow for faster detection of *Legionella* than the primers disclosed in other known assays, such as that of US patent publication No. 2012071661.

In order to further understand the above-described device and to demonstrate how the method may be carried out in practice, certain embodiments will now be described with reference to examples described below. The following examples are provided for illustration and do not limit the disclosure or scope of the claims and specification.

### EXAMPLE 1: IDENTIFICATION OF TARGET DNA AND ASSAYS USED

The inventors a Legionella gene identified as *lpg1974* (Weissenmayer, Prendergast et al. 2011) as a source of a suitable target for novel Legionella detection reagents. This gene, also known as *ompS* (NCBI Genbank Accession No. M76178.1) was found to be fairly stably expressed at relatively high levels to other Legionella genes. Thus, this gene and its encoding outer membrane protein met the inventors' twin goals of a nucleotide sequence whose expression would be insensitive to growth conditions and unique to *Legionella* to prevent detection of non-target organisms.

### A. Developing and Testing qPCR Primers

Because the 5'end of *ompS* appeared to be less variable relative to other regions of the gene and appeared to be more specific to *L. pneumophila* organisms, it was targeted for qPCR primer generation. The inventors employed a tool, i.e., the IDT primer design website (http://www.idtdna.com/scitools/Applications/RealTimePCR/) to construct qPCR primers with specific melting temperature, length, and homodimer/heterodimer features (Table 1).

Amplification of target DNA was done under the following conditions:
95°C for 3 minutes; followed by 41 cycles of: 95°C for 10 seconds, 58°C for 10 seconds, 72°C for 30 seconds; then 95°C for 10s, followed by a melt curve using software default conditions. qPCR amplification was done in the CFX96 Bio-Rad System with a commercially purchased *Legionella* genomic standard used for run validation. The qPCR conditions varied from the recommended instructions, but as efficiency and R^2 values were within normal parameters, it is unlikely that the changes had any effect. These conditions involved using per reaction well 12.5 µl Bio-Rad iQ SYBR Master Mix, 0.15 µl of both forward and reverse primer (stock primer concentration was 50 µM), and 4.7 µl molecular-biology grade H₂O (instead of the correct 7.2 µl H₂O). To that volume 5 µl of 1/100 diluted purified genomic DNA was added.

### B. Developing PCR Primers

Regions of DNA that could be used either for traditional PCR approaches or a hybridization-based approach were also analyzed for a broader detection, e.g., of multiple Legionella species. To do this, the DNA sequence of the *ompS* gene from *L. pneumophila* and the ompS gene from *L. longbeachae* were aligned and directly compared to identify regions of total similarity or sufficiently high sequence similarity to permit a primer with 1-2 wobble bases to be identified. "Wobble bases" refer to certain nucleotide bases located in a primer sequence for which alternative base options are possible, based upon the primer pool used in a reaction. For example, a wobble base of "R" would be 50% guanine and 50% cytosine in a given primer pool. This approach allowed for the broadening of a primer reactivity to include highly related but not 100% identical regions of DNA.

The *ompS* alignment was visually examined to identify 4 regions of DNA that could be used for primers, with complementary primers generated so that key regions could be used for different tests. The primers identified in Table 1 below, were tested using a traditional PCR 2X Master Mix (Promega) with the following conditions: 12.5 µl Master Mix, 0.5 µl of both forward and reverse primers, and 9.5 µl dH₂O. To this, 2 µl of purified genomic DNA isolated using the MolBio Ultraclean DNA Isolation Kit was added. The PCR conditions in the initial screen were as follows: 2 min at 95°C, 35 cycles of: 30s at 95°C, 30s at 45°C (40°C for primers 528 SEQ ID NO: 4 and 825Gc SEQ ID NO: 10 due to primer melting temperature requirements), and 30s at 72°C; then 10 min at 72°C for a final extension step.

The primer sequences SEQ ID NO: 1-10 and known sequences SEQ ID NO: 15 and 16 identified in Table 1 are located on the ompS gene.

**TABLE 1: PRIMER SEQUENCES AND NAMES**

| Inventor's Primer Sequences | Sequence⁴ | Target | Appln | SEQ ID # |
|---|---|---|---|---|
| Legionella OmpS Set 3 Forward | GCA GTG CTT TGT TTG CAG GTA CGA | ompS | qPCR | 1 |
| Legionella OmpS Set 3 Reverse | ATG CCA ATT TCT CCA GCC ACC AAC | ompS | qPCR | 2 |
| OmpS 327F | TTT GCA GGT ACS ATG GGT CCA GT | ompS | PCR | 3 |
| OmpS 528F | GAA GGT TCT TAT CAC TTC AAC AC | ompS | PCR | 4 |
| OmpS 660F | GAA MTG GGK CAA TTY GTT GAT | ompS | PCR | 5 |
| OmpS 825F | ATG AAY TAT GTA TTY GGT AA | ompS | PCR | 6 |
| GC-OmpS 327F⁵ | ACT GGA CCC ATS GTA CCT GCA AA | ompS | PCR | 7 |
| GC-OmpS 528F⁵ | GTG TTG AAG TGA TAA GAA CCT TC | ompS | PCR | 8 |
| GC-OmpS 660F⁵ | ATC AAC RAA TTG MCC CAK TTC | ompS | PCR | 9 |
| GC-OmpS 825F⁵ | TTA CCR AAT ACA TAR TTC AT | ompS | PCR | 10 |
| Primer Sequences obtained from other publications | | | | |
| mompS-492F¹ | GACATCAATGTGAACTGG | mompS | PCR | 11 |
| mompS-1116R¹ | TGGATAAATTATCCAGCCGGACTTC | mompS | PCR | 12 |
| mompS-450F² | TTG ACC ATG AGT GGG ATT GG | mompS | PCR | 13 |
| mompS-1126R² | TGG ATA AAT TAT CCA GCC GGA CTT C | mompS | PCR | 14 |
| US20120171661F³ | gcg gct gta ttt gct ctg gga a | ompS | PCR | 15 |
| US20120171661R³ | taa gcc tat gta ggg gcc aga tgc | ompS | PCR | 16 |

| | | | | |
|---|---|---|---|---|
| ¹Source is reference no. 11. ²Source is reference no. 10 ³ Source is reference no. 8. ⁴ Nucleic acid "wobble base" abbreviations include S representing either C or G; M representing either A or C; Y representing either C or T; R representing either A or G; and K representing either GorT. ⁵ The "gc" on the primer name refers to "generated complement", where the primer manufacturer (IDT) generated a primer that was located on the same location on the opposite strand of DNA so that the same region of DNA could be tested with primers both 5' and 3' to its location. | | | | |

### C. Initial Primer Comparison

A series of benchmarking of known primers was conducted to identify whether the novel *ompS* primers demonstrated an improvement when compared with other known *ompS* primers. In one comparison at conditions that were best suited for the primer setup (2°C below the primer melting temperature (TM), the reaction conditions were 12.5 µl Master Mix, 0.5 µl of both forward and reverse primers, and 10.5 µl dH₂O where 1 µl of purified genomic DNA isolated using the MolBio Ultraclean DNA Isolation Kit was added. DNA from defined strains and from nucleic acid isolated from a CDC ELITE testing panel were assayed.

### D. Refined Primer Comparison

Cell suspensions from defined bacterial species (*Legionella pneumophila* ATCC BAA74, *P. Pseudomonas aeruginosa* ATCC 15442, *Burkolderia cepacia* ATCC 25416, *Sphingomonas paucimobilis* BAA1092, *Flavobacterium odoratum* (aka *Myroides odoratus*) NCIMB 13294, *Enterobacter aerogenes* ATCC 13048, and *Klebsiella pneumonia* ATCC 8308) were obtained and split into two aliquots. One aliquot was plated to obtain a cfu/ml count as a measure of organism concentration and the other was subjected to DNA isolation via the MolBio UltraClean DNA Kit. Once the cfu/ml of each starting colony was identified, each DNA vial was assigned an equivalent genomic unit/ml concentration based on the cell counts. From that, a dilution range was generated to simulate absence of target organism, then increasing amounts of *Legionella* relative to a high level of non-target organism DNA (see conditions specified in Table 2).

**TABLE 2: PRIMER EXPERIMENTAL CONDITIONS**

| Test | Primer Sequences | Target | Rxn Mix | PCR Cycle Conditions (DNA in water; mimics low concentration samples) |
|---|---|---|---|---|
| qPCR test | SEQ ID NOs: 1 and 2 | ompS | qPCR | 0.333µM primer (ideally 0.3µM); 95°C for 3 min, 41 cycles of denaturation for 10 sec at 95°C; annealing for 10 sec at 58°C; elongation for 30 sec at 72°C; 10 sec at 95°C; a melt curve analysis SYBR green detection |
| Primer Conditions | SEQ ID NOs: 4 and 10 | ompS | PCR | 1µM primer, 3 min at 95°C; 35 cycles of denaturation for 30 sec at 94°C; annealing for 30 sec at 40°C; elongation for 30 sec at 72°C; 10 min 72°C extension step |
| Compariso n Test 1 | SEQ ID NOs: 11 and 12 | momp S | PCR | 1µM primer, 35 cycles of denaturation for 30 sec at 94°C; annealing for 30 sec at 50°C; elongation for 30 sec at 72°C; 10 min 72°C extension step |
| Compariso n Test 2 | SEQ ID NOs: 13 and 14 | momp S | PCR | 0.2µM primer; 35 cycles of denaturation for 30 sec at 95°C; annealing for 30 sec at 55°C; elongation for 40 sec at 72°C; 10 min 72°C extension step |
| Compariso n Test 3 | SEQ ID NOs: 15 and 16 | ompS | PCR | 1µM primer; 35 cycles of denaturation for 60 sec at 94°C; annealing for 60 sec at 58°C; elongation for 90 sec at 72°C; 10 min 72°C extension step |

A master block was made with the DNA concentrations so that the same DNA material would be used in each reaction. To confirm that no meaningful changes in the DNA sample or that false-positives occurred from cross-contamination from the start of the reaction to the end, the primers SEQ ID NOs: 1-10 were tested at the beginning and towards the end of the experiment. Primers were tested at the conditions suggested in other publications.

### E. DNA Gel Protocol

To visualize PCR fragments either Lonza's Flashgel cassette-based system or a standard operating procedure was followed for gel casting, buffer, and safety considerations. According to the SOP, 5µl of PCR product/loading dye was added to each well with 5 of 100bp DNA Ladder (Promega) /loading dye added to the left most well (2µl for the Lonza Flashgel system) . 1.5% agarose gels were run in 1x TBE buffer for 30 minutes at 100V. Then, they were placed into an empty plastic pipette tip box and exposed to 50 ml of 1X SYBR Green (Invitrogen) for approximately 60 minutes, then visualized on the Bio-Rad Gel XR Imager. Lonza Flashgels were operated using manufacturer's instructions and visualized with the built-in imaging camera.

### EXAMPLE 2: qPCR-BASED METHOD FOR BROAD LEGIONELLA DETECTION

qPCR primers SEQ ID NOs. 1 and 2 were generated to determine whether the *ompS* gene could be used in a qPCR-based method for broad *Legionella* detection. DNA from liquid culture-grown *P. aeruginosa* and *P. denitrificans* was used as negative controls. DNA from *L. pneumophila* BAA74 served as a positive control, with DNA extracted from strains isolated from CDC ELITE test samples (*L. dumoffii, L. longbeachae, L. feelei, and L. cherii*) used as experimental samples. To obtain *Legionella* DNA, colonies were swabbed from bacterial media plates and agitated into water within a biosafety cabinet for extraction using the MoBio Ultraclean DNA extraction kit. DNA was diluted 1/100 in dH₂O prior to analysis and with plastic reagents from Bio-Rad used in the qPCR run. Commercially purchased *L. pneumophila* genomic standards served as the material for the 5-log standard dilutions.

Efficiency of the run was 95.7% with an R^2 of 0.999, suggesting that despite a slightly higher concentration of mix and primers, the run was well-within acceptable qPCR norms. *L. pneumophila* and *L. dumoffii* appeared to be preferentially amplified relative to other *Legionella* bacterial samples. Negative controls *P. denitrificans* and a non-template added control failed to have meaningful amplification, with *P. aeruginosa* amplified at 1x 10¹ cfu/ml range. Given that the cells used in the extraction were straight from late exponential/stationary phase cells, this count is likely not an accurate measurement of *P. aeruginosa* cell presence, but rather indicative of low-level cross contamination. Of particular interest is the high reactivity of *L. dumoffii* with the qPCR *ompS* primers.

Repetition of this experiment involves normalizing the DNA input following enumeration of live cells to get approximately equivalent genomic units/ml and assist in confirming that this high degree of similarity is accurate.

### EXAMPLE 3: HYBRIDIZATION METHOD FOR BROAD LEGIONELLA DETECTION

Following what observed to be a highly selective *Legionella* primer design and a determination that primers (Table 1) should be spaced further apart for the proposed hybridization approach, new primers were selected from a more central to 3' end of the *ompS* gene. Specific regions were isolated based on high degree of sequence homology to *L. longbeachae.* The "gc" on the primer name refers to "generated complement", where the primer manufacturer (IDT) generated a primer that was located on the same location on the opposite strand of DNA so that the same region of DNA could be tested with primers both 5' and 3' to its location. The suitability of each primer set was then tested using the conditions listed in Table 2.

To test these primers, genomic DNA from swabbed *Legionella* strains (or liquid cultures in the case of *P. aeruginosa* and *P. denitrificans*) were assayed with the *ompS* primers 327, 528, 660, and 825 along with their "gc" counterparts (SEQ ID NOs: 3-10, respectively). The melting temperature for each reaction was 45°C or 40°C depending on primer melting temperature requirements. For this initial testing reaction conditions were as follows: 1µM primer, 3 min 95°C, 35 cycles of denaturation for 30 s at 94°C; annealing for 30 s at 40°C or 45°C, and elongation for 30 s at 72°C with a 10 min 72°C extension step at the end. Given the cleanliness of the reactions, the primer pair 528/825gc SEQ ID NOs: 4 and 10 were selected for additional analysis.

Once these reactions had been done, additional work to compare these primers to other known sequences, e.g., primers SEQ ID NOs: 15 and 16 (ref. 8) were performed to determine whether the primers SEQ ID NO: 4 and 10 provided an improvement in *Legionella* detection. The testing fell within two approaches- testing of primers against purified genomic DNA and testing of mock-samples provided by the US CDC for ELITE certification testing. Improvement with the inventors' primers was unclear when assaying the CDC ELITE samples. However, when the same test was run against purified DNA, there was an improvement observed in cross-reactivity. Primers 528 and 825gc showed less cross-reactivity with non-Legionella microbial contamination, e.g., *P. aeruginosa,* that the other sequences known in the art.

Following this initial screen, the testing protocol was amended. The test concentrations of DNA with the defined genomic DNA may have been too high to accurately determine if there was an improvement with the inventors' primers relative to the primers SEQ ID NOs. 15 and 16. Based on interactions with cooling water services companies and field trial experiences, typical bacterial burden in these samples tended to be around 1x10⁵ cfu/ml. Second, the primer pool was extended to include two other sets of known primers from references 10 and 11, SEQ ID NOs: 11 through 14 of Table 1. Specific microbial species were tested based on publications (see e.g., refs. 1-3) and discussions with water treatment applications specialists, and availability of these contaminating microbial strains.

A master block of isolated DNA with the equivalent genomic unit/ml to counted cfu/ml was generated as described in Example 1 and used for PCR analysis. The degree to which cross-reactivity was observed in either total non-target samples or samples with a high non-target to *L. pneumophila* DNA ratio was then observed. The 528F and 825gc reverse primers were tested at the beginning and end of the experiment to ensure that any cross-reactivity observed with other primers were genuine and not due to inadvertent cross-contamination of material. The first round of primer testing resulted in gels too faint to read clearly.

Interestingly, a non-specific amplification was observed with these primers against *B. cepacia* and *E. aerogenes,* which have been highlighted with a box. These results immediately suggest that the 528F and 825gc primers constitute an improvement over the known primers. A similar improvement was not observed with the *K. pneumonia* tests.

The known 1126R/450 primers SEQ ID NOs. 14 and 13 were assayed according to the conditions listed in Table 2. Although data on the performance of the 1126R/450 primers against *Klebsiella* were absent, there was not a clear diminution of performance relative to the 528F/825gc primers with the species tested with regards to cross-reactivity. The primers appeared to have a higher threshold for detection (1x10²-1x10³) of *Legionella.* Some wells had failure to amplify; therefore this experiment when repeated is anticipated to confirm a detection sensitivity improvement.

Finally, the primers SEQ ID NO: 15 and 16 were tested per conditions in Table 2 to determine performance relative to the inventors' primers.

In summary, while the qualities of gels were initially poor and will be repeated, there was a clear cross-reactivity observed against *B. cepacia.* Again, this suggests that the DMC primers have an improvement relative to the US20120171661 primers.

However, the present data on these primers provides evidence indicating that the inventor-identified primers constitute an improvement of other *ompS* or *mompS*-focused primers for the detection of *Legionella* in a complex sample.

It should be understood that while various embodiments in the specification are presented using "comprising" language, under various circumstances, a related embodiment is also be described using "consisting of' or "consisting essentially of' language. It is to be noted that the term "a" or "an", refers to one or more, for example, "a test compound," is understood to represent one or more test compounds. As such, the terms "a" (or "an"), "one or more," and "at least one" is used interchangeably herein.

## Claims

1. A reagent for examining a water supply for microbial contamination comprising a set of primers comprising a forward nucleotide sequence primer comprising SEQ ID NO: 4 and a reverse nucleotide sequence primer comprising SEQ ID NO: 10, the nucleotide sequences having high specificity for a *Legionella* microbial species, wherein said nucleotide sequences do not cross-react, or minimally cross- reacts, with a microbial species other than *Legionella.*

2. The reagent according to claim 1, wherein the set of primers further comprises a forward primer of SEQ ID NOs: 1, 3, 5 or 6 and a reverse primer of SEQ ID NOs: 2, 7, 8 or 9.

3. The reagent of claim 1, further comprising primers SEQ ID Nos: 1, 2, 3, 5, 6, 7, 8 and 9.

4. The reagent according to any preceding claim, which further comprises a substrate upon which one or more of the nucleotide sequences are immobilized or fixed.

5. The reagent according to any preceding claim, wherein a detectable label or label component is associated with at least one primer.

6. The reagent according to claim 4, wherein said substrate is an array, a microarray, a microchip, a plastic surface, or a glass surface.

7. A kit comprising the reagent of any preceding claim and one or more components selected from a label, a substrate, a label component capable of interacting with the label and generating a detectable signal.

8. A water monitoring device comprising a reagent of claim 1 to 6.

9. A method of examining a water supply for microbial contamination comprising:
(i) contacting a water supply with a reagent according to any one of claims 1 to 6 in order to amplify a target sequence with a high degree of target specificity in polymerase chain reaction (PCR) or real time qualitative polymerase chain reaction (qPCR) methodologies; and
(ii) detecting, or measuring, a contaminating concentration of a *Legionella* species in the water supply.

10. The method according to claim 9, wherein the PCR or qPCR comprises annealing the set of primers to a targeted nucleic acid sequence present in *Legionella* at a selected annealing temperature lower than 58° C.

11. The method according to claim 9, comprising distinguishing between contamination with *Legionella* species from contamination with a *Pseudomonas* species.

## Patentansprüche

1. Ein Reagens zum Untersuchen einer Wasserversorgung auf mikrobielle Verunreinigung, beinhaltend einen Satz Primer, beinhaltend einen Vorwärtsnukleotidsequenzprimer, beinhaltend SEQ ID NO: 4, und einen Rückwärtsnukleotidsequenzprimer, beinhaltend SEQ ID NO: 10, wobei die Nukleotidsequenzen eine hohe Spezifität für eine mikrobielle Spezies *Legionella* aufweisen, wobei die Nukleotidsequenzen mit einer mikrobiellen Spezies außer *Legionella* nicht kreuzreagieren oder minimal kreuzreagieren.

2. Reagens gemäß Anspruch 1, wobei der Satz Primer ferner einen Vorwärtsprimer der SEQ ID NOs: 1, 3, 5 oder 6 und einen Rückwärtsprimer der SEQ ID NOs: 2, 7, 8 oder 9 beinhaltet.

3. Reagens gemäß Anspruch 1, das ferner Primer der SEQ ID Nos: 1, 2, 3, 5, 6, 7, 8 und 9 beinhaltet.

4. Reagens gemäß einem der vorhergehenden Ansprüche, das ferner ein Substrat beinhaltet, auf dem eine oder mehrere der Nukleotidsequenzen immobilisiert oder fixiert sind.

5. Reagens gemäß einem der vorhergehenden Ansprüche, wobei eine nachweisbare Kennzeichnung oder Kennzeichnungskomponente mit mindestens einem Primer assoziiert ist.

6. Reagens gemäß Anspruch 4, wobei das Substrat ein Array, ein Microarray, ein Mikrochip, eine Kunststoffoberfläche oder eine Glasoberfläche ist.

7. Ein Kit, beinhaltend das Reagens gemäß einem der vorhergehenden Ansprüche und eine oder mehrere Komponenten, ausgewählt aus einer Kennzeichnung, einem Substrat, einer Kennzeichnungskomponente, die mit der Kennzeichnung interagieren kann und ein nachweisbares Signal erzeugt.

8. Eine Vorrichtung zum Überwachen von Wasser, die ein Reagens gemäß Anspruch 1 bis 6 beinhaltet.

9. Ein Verfahren zum Untersuchen einer Wasserversorgung auf mikrobielle Verunreinigung, beinhaltend:
(i) In-Kontakt-Bringen einer Wasserversorgung mit einem Reagens gemäß einem der Ansprüche 1 bis 6, um eine Zielsequenz mit einem hohen Grad an Zielspezifität der Methodologie Polymerase-Kettenreaktion (PCS) oder qualitative-Polymerase-Kettenreaktion-in-Echtzeit (qPCR) zu amplifizieren; und
(ii) Nachweisen oder Messen einer verunreinigenden Konzentration einer *Legionella-*Spezies in der Wasserversorgung.

10. Verfahren gemäß Anspruch 9, wobei die PCR oder qPCR das Annealing des Satzes Primer bei einer ausgewählten Annealing-Temperatur von weniger als 58 °C an einer in Legionella vorhandenen Ziel-Nukleinsäuresequenz beinhaltet.

11. Verfahren gemäß Anspruch 9, das das Unterscheiden zwischen Verunreinigung mit Legionella-Spezies und Verunreinigung mit einer Pseudomonas-Spezies beinhaltet.

## Revendications

1. Un réactif pour l'examen d'une alimentation en eau à l'égard d'une contamination microbienne comprenant un ensemble d'amorces comprenant une amorce avant de séquence nucléotidique comprenant la SEQ ID N° : 4 et une amorce arrière de séquence nucléotidique comprenant la SEQ ID N° : 10, les séquences nucléotidiques disposant d'une spécificité élevée à l'égard d'une espèce microbienne *Legionella,* lesdites séquences nucléotidiques n'ayant pas de réaction croisée, ou ayant des réactions croisées imperceptibles, avec une espèce microbienne autre que la *Legionella.*

2. Le réactif selon la revendication 1, dans lequel l'ensemble d'amorces comprend en outre une amorce avant de SEQ ID N° : 1, 3, 5 ou 6 et une amorce arrière de SEQ ID N° : 2, 7, 8 ou 9.

3. Le réactif de la revendication 1, comprenant en outre des amorces de SEQ ID N° : 1, 2, 3, 5, 6, 7, 8 et 9.

4. Le réactif selon n'importe quelle revendication précédente, qui comprend en outre un substrat sur lequel une ou plusieurs des séquences nucléotidiques sont immobilisées ou fixées.

5. Le réactif selon n'importe quelle revendication précédente, dans lequel une étiquette détectable ou un constituant d'étiquette est lié(e) à au moins une amorce.

6. Le réactif selon la revendication 4, dans lequel ledit substrat est un réseau, un microréseau, une micropuce, une surface en plastique, ou une surface en verre.

7. Un kit comprenant le réactif de n'importe quelle revendication précédente et un ou plusieurs constituants sélectionnés parmi une étiquette, un substrat, un constituant d'étiquette apte à interagir avec l'étiquette et générer un signal détectable.

8. Un dispositif de surveillance d'eau comprenant un réactif de la revendication 1 à 6.

9. Une méthode d'examen d'une alimentation en eau à l'égard d'une contamination microbienne comprenant :
(i) la mise en contact d'une alimentation en eau avec un réactif selon n'importe laquelle des revendications 1 à 6 afin d'amplifier une séquence cible avec un degré élevé de spécificité cible dans les méthodologies de réaction en chaîne par polymérase (PCR) ou de réaction en chaîne par polymérase qualitative en temps réel (qPCR) ; et
(ii) la détection, ou la mesure, d'une concentration contaminante d'une espèce *Legionella* dans l'alimentation en eau.

10. La méthode selon la revendication 9, dans laquelle la PCR ou qPCR comprend l'annelage de l'ensemble d'amorces à une séquence d'acide nucléique ciblée présente dans la *Legionella* à une température d'annelage sélectionnée inférieure à 58 °C.

11. La méthode selon la revendication 9, comprenant le fait de faire la distinction entre une contamination par l'espèce *Legionella* et une contamination par une espèce *Pseudomonas.*
